# FASCICULE DE BREVET EUROPEEN

(11) **EP 2 039 356 B1**
(45) Date de publication et mention de la délivrance du brevet: **10.07.2013**
(21) Numéro de dépôt: 08368017.3
(22) Date de dépôt: 12.09.2008
(51) Int. Cl.: A61K 31/221, A61K 31/395, A61K 31/4178, A61K 31/475, A61K 31/7048, A61K 45/06, A61P 43/00

(54) **Composition sialagogue à base d'un agoniste muscarinique M3 et d'un antagoniste adrénergique alpha-2**
Den Speichelfluss anregendes Stoffgemisch enthaltend einen Muskarin-M3-Agonisten und einen Alpha-2- adrenergen Antagonisten
Sialagogue composition comprising a M3 muscarinic agonist and an alpha-2 adrenergic antagonist

(30) Priorité: 20.09.2007 FR 0706596
(43) Date de publication de la demande: 25.03.2009
(73) Titulaire: Universite De Nice-Sophia Antipolis, 06103 Nice Cedex 02 (FR); Centre Hospitalier Universitaire De Nice, 06003 Nice Cedex 01 (FR)
(72) Inventeur: Madinier, Isabelle, 06000 Nice (FR)
(74) Mandataire: Macquet, Christophe

(56) Documents cités:
- US-A1- 2007 128 284
- COPPES R P ET AL: "Muscarinic receptor stimulation increases tolerance of rat saliva gland function to radiation damage" INTERNATIONAL JOURNAL OF RADIATION BIOLOGY TAYLOR & FRANCIS UK, vol. 72, no. 5, novembre 1997 (1997-11), pages 615-625, XP009097905 ISSN: 0955-3002
- DATABASE CA [Online] CHEMICAL ABSTRACTS SERVICE, COLUMBUS, OHIO, US; 16 décembre 2001 (2001-12-16), OKAMURA, Y.: "Salivary secretion" XP002474381 extrait de STN Database accession no. 1942:44517 & OKAYAMA IGAKKAI ZASSHI , 51, 371-82;IN GERMAN, 383 CODEN: OIZAAV; ISSN: 0030-1558, 1939,
- DATABASE BIOSIS [Online] BIOSCIENCES INFORMATION SERVICE, PHILADELPHIA, PA, US; 1997, BAGHERI H ET AL: "A comparative study of the effects of yohimbine and anetholtrithione on salivary secretion in depressed patients treated with psychotropic drugs" XP002511099 Database accession no. PREV199799745848 & EUROPEAN JOURNAL OF CLINICAL PHARMACOLOGY, vol. 52, no. 5, 1997, pages 339-342, ISSN: 0031-6970
- DATABASE MEDLINE [Online] US NATIONAL LIBRARY OF MEDICINE (NLM), BETHESDA, MD, US; janvier 1996 (1996-01), "First effective drug for dry mouth." XP002511100 Database accession no. NLM11363126 & POSITIVELY AWARE : THE MONTHLY JOURNAL OF THE TEST POSITIVE AWARE NETWORK 1996 JAN-FEB, vol. 7, no. 1, janvier 1996 (1996-01), page 6, ISSN: 1523-2883

## Description

L'invention concerne une composition sialagogue. Elle concerne en outre cette composition pour son utilisation pour augmenter la sécrétion salivaire, ainsi que pour le traitement d'un défaut de la fonction salivaire tel que l'hyposialie et la xérostomie.

L'hyposialie est une diminution de la fabrication de salive par les glandes salivaires (au repos et stimulée par l'alimentation), qui provoque une sécheresse buccale. L'asialie est une absence totale de sécrétion de salive. La xérostomie est une forme de sécheresse excessive de la bouche, secondaire à une hyposialie ou à une asialie.

Les principales causes d'hyposialies sont les syndromes secs (maladies autoimmunes), l'infection chronique par le virus de l'hépatite C, la destruction des glandes salivaires par irradiation lors des chimiothérapies des cancers de la tête du cou, et de nombreux médicaments, en particulier les anticholinergiques et les psychotropes.

Les traitements médicamenteux connus des hyposialies, de l'asialie et/ou de la xérostomie à visée étiologique comprennent les composés sialagogues par administration orale : Salagen^{®} (pilocarpine, comprimé enrobé), Bisolvon^{®} (bromhexine chlorhydrate), Sulfarlem S 25^{®} (anétholtrithione).

On connaît également dans l'art antérieur les documents Coppes et al., International Journal of Radiation Biology vol.72, no. 5, 615-625, nov 1997, qui décrit l'administration intrapéritonéale de métacholine et de réserpine l'une après l'autre au sein de compositions distinctes chez des rats avec un effet sur le débit salivaire de la glande sous-mandibulaire, Okamura, Chemical Abstract Service, 16 dec 2001, qui décrit que la sécrétion salivaire est augmentée par l'injection de pilocarpine ou de yohimbine dans les vaisseaux sanguins d'un chien atteint de fistules permanentes des glandes parotide et sous-maxillaire, ou encore US2007128284 qui décrit la pilocarpine et la yohimbine parmi les composés pour traiter la xérostomie, Bagheri et al., European Journal of Clinical Pharmacology, vol. 52, no. 5, 339-342, 1997, qui décrit l'administration orale de yohimbine pour le traitement de la xérostomie et Bethesda, Positively aware : the monthly journal of the test positive aware network, vol. 7, no. 1, jan 1996, qui décrit l'utilisation périphérique de pilocarpine pour le traitement de la xérostomie.

Les traitements à visée symptomatique comprennent :
- des composés sialagogues issus de la phytothérapie ;
- des substituts salivaires ou protecteurs en spray type Artisial^{®} ou Aequasyal^{®};
- des produits d'hygiène bucco-dentaires spécifiques : Aequasyal^{®}, Bioxtra^{®}, Biotène^{®}, Orajel^{®}, Oasis^{®} etc.

Les tentatives d'amélioration des hyposialies sévères comprennent 1) l'hydratation générale et locale, 2) l'hygiène buccodentaire, 3) les soins de bouche antiseptiques, 4) la salive artificielle, 5) les sialagogues centraux, 6) les antifongiques, 7) les antalgiques (avant les repas).

Actuellement, aucun de ces traitements n'apparaît satisfaisant, car ils ne permettent pas de soulager efficacement les individus atteints d'un défaut de la fonction salivaire.

La salive est élaborée par des glandes exocrines : les glandes principales, parotides, sous-maxillaires et sub-linguales (stimulées par l'alimentation), et les glandes accessoires disséminées sous les muqueuses buccales (salive « de repos »).

La salivation est une réponse réflexe (i.e. indépendante du contrôle de la volonté) qui résulte de phénomènes réflexes, automatiques ou conscients. Elle fait intervenir un système complexe avec :
- divers récepteurs au niveau de la bouche, dont la stimulation active des voies nerveuses afférentes (médiateur : glutamate) de la bouche vers le système nerveux central (SNC) ;
- l'intégration dans le SNC des stimulus buccaux avec d'autres données, issues de l'olfaction, de la vue, de l'ouïe et de la mémoire ;
- l'activation de voies nerveuses efférentes du SNC vers les glandes salivaires, parasympathiques activatrices (médiateur acétylcholine) et sympathiques inhibitrices (médiateur adrénaline et noradrénaline) avec deux types de récepteurs au niveau de la cellule sécrétrice salivaire.

Ce système comprend notamment :
- des stimulations « périphériques A », telles que « douleur-mouvement » (capteurs sur les neurones ascendant dans la muqueuse buccale) et « goût » (récepteurs sur les cellules gustatives : récepteurs sensitifs [sensibilité tactile, chaud, froid, douleur, piquant, astringence, CO2] et récepteurs sensoriels [goûts acide, salé, amer, sucré, aromatique, unami]);
- des stimulations « cérébrales », les voies efférentes parasympathiques et sympathiques partant du cerveau. Les neurones parasympathiques sont stimulés par la fixation d'acétylcholine sur leurs récepteurs M3 postynaptiques ; ils sont inhibés par la fixation d'adrénaline ou de noradrénaline sur leurs hétérorécepteurs α2 présynaptiques. Schématiquement, la noradrénaline est sécrétée de façon basale par les neurones sympathiques, et l'adrénaline est libérée en cas de stress par les glandes médullo-surrénales.
- des stimulations « périphériques B » dans les glandes salivaires, où les récepteurs M3 postsynaptiques sont portés par les cellules sécrétrices salivaires, et les hétérorécepteurs α2 présynaptiques par le dernier neurone de la chaîne parasympathique. Dans les glandes salivaires, l'acétylcholine a aussi un effet activateur en se fixant sur les récepteurs de type M2 sur les cellules musculaires lisses de type digestif (contraction des glandes et expulsion de salive). Adrénaline et noradrénaline ont des effets complexes sur les muscles vasculaires lisses.

La Demanderesse a réalisée une étude clinique non publiée sur 120 sujets polymédicamentés. De façon surprenante, cette étude a montré que les causes réelles des hyposialies médicamenteuses sont :
- l'usage d'anticholinergiques « durs » : les anticholinestérasiques en particulier les antiparkinsoniens du type tropatépine (Lepticur^{®}), l'oxybutynine (Ditropam^{®}) contre l'incontinence urinaire, les spécialités antiasthmatiques contenant de l'ipratropium bromure (Atrovent^{®}, Bronchodual^{®}) et un psychotrope phénothiazique ayant un effet secondaire anticholinergique important connu, la cyamémazine (Tercian®);
- les médicaments susceptibles d'altérer le biofilm oral, tels que les antibiotiques et les corticoïdes, et les bains de bouche antiseptiques ;
- le nombre de médicaments (principes actifs) pris chaque jour, mais ce paramètre est significativement associé à la prise d'anticholinergiques « durs » (avec une différence statistiquement significative entre le groupe de patients qui ne souffre pas de sècheresse buccale et qui prend en moyenne 6,3±3,3 principes actifs par jour et le groupe de patients qui souffre de sècheresse buccale et qui prend en moyenne 9,2±4,0 principes actifs par jour : p=0,003) et les bains de bouche ; le rôle des excipients des médicaments dans la sécheresse buccale reste à évaluer.

L'analyse statistique des résultats n'a pas montré d'impact des autres psychotropes sur la sécheresse buccale, ni d'autres médicaments.

Des recherches chez le rat ont porté sur l'effet d'un antagoniste adrénergique α2 tel que la Yohimbine (Y) sur l'activation de salivation induite par un agoniste muscarinique M3 tel que la Pilocarpine (P). Les résultats de ces recherches (1. Dos Santos Moreira T et al. Arch Oral Biol 47:429-434, 2002 ; 2. Takakura AC et al Brain Res Bull, 30:383-386, 2003.) décrivent que P a un effet inducteur de la salivation, et que Y présente un effet activateur de la salivation induite par P, lorsque Y est administré de manière centrale (i.e. pour une action dans le cerveau) et P est administré de manière centrale ou périphérique (injection intra péritonéale).

Ces résultats ne fournissent pas de solution pour un traitement satisfaisant d'un défaut de la salivation chez un individu. En effet, d'après ces articles, Y est administré de manière centrale pour agir au niveau du cerveau. Or ce mode d'administration est invasif et agressif et ne convient pas pour traiter facilement et efficacement le défaut de salivation chez un individu. Par conséquent, ces données ne suggèrent pas de solution pour un traitement plus efficace du défaut de la salivation et facile à mettre en oeuvre.

Compte tenu de ce qui précède, un problème que se propose de résoudre l'invention est d'obtenir un traitement amélioré, plus efficace et facile d'utilisation d'un défaut de la fonction salivaire chez un individu qui en est atteint.

La solution proposée de l'invention à ce problème a pour premier objet une composition sialagogue comprenant, dans un véhicule pharmaceutiquement acceptable convenant pour une administration périphérique, un agoniste muscarinique M3 choisi parmi la pilocarpine ou le chlorhydrate de pilocarpine, caractérisée en ce qu'elle comprend en outre un antagoniste adrénergique α 2 choisi parmi la yohimbine ou le chlorhydrate de yohimbine.

Elle a pour second objet l'utilisation d'une composition selon l'invention pour l'obtention d'un médicament pour le traitement d'un défaut de la fonction salivaire.

D'autres recherches chez le rat ont porté sur l'effet de la Yohimbine (Y) (antagoniste adrénergique α 2) sur l'activation de salivation induite par la Pilocarpine (P) (agoniste muscarinique M3). Les résultats de ces recherches (3. Cecanho R et al. J Auton Nerv Syst 76:9-14, 1999. 4. Saad WA et al. Auton Neurosci 112:31-36, 2004) décrivent que P a un effet inducteur de la salivation, et que Y présente :
- un effet inhibiteur de la salivation induite par P, lorsque Y est administré de manière périphérique (injection intra péritonéale) et P est administré de manière centrale (réf. 3) ; et
- aucun effet modifiant la salivation induite par P, lorsque Y et P sont administrés de manière périphérique (injection intra péritonéale) (réf. 4).

Dans le cadre de ces recherches, l'administration centrale est une administration i.c.v. (intra-cérébro-ventriculaire), LV (ventricule latéral) ou MSA (aire septale médiane), et l'administration périphérique est une administration par voie orale (p.o.) ou intrapéritonéale (i.p.).

Ces articles chez le rat ne divulguent ni ne suggèrent une composition comprenant à la fois P et Y ni a fortiori une telle composition apte à être administrée par voie périphérique, en particulier par voie orale. En effet, dans ces recherches, P et Y sont administrés l'un après l'autre et non au sein d'une même composition. De plus, ils sont administrés séparément de manière centrale ou intra péritonéale, mais aucune administration par voie orale n'est décrite.

De plus, d'après l'article de réf. 4, lorsque Y et P sont administrés chacun de manière périphérique (injection intra péritonéale), il n'est pas observé de modification de la salivation induite par P seule. Par conséquent, cet enseignement ne suggère pas qu'une association d'un antagoniste adrénergique α2 tel que Y et d'un agoniste muscarinique M3 tel que P administrée par voie périphérique améliorerait la salivation induite par P seule. Au contraire, cet enseignement éloigne de la solution selon l'invention, en établissant un préjugé selon lequel une telle association ne fournit pas un traitement efficace d'un défaut de la salivation.

Pourtant, de manière surprenante, la Demanderesse a mis en évidence chez l'homme qu'une association d'un antagoniste adrénergique α2 et d'un agoniste muscarinique M3 au sein d'une même composition améliore de manière significative et synergique la salivation induite par l'agoniste muscarinique M3 seul lorsque cette association est administrée par voie orale. En effet, les expérimentations par voie intrapéritonéale chez le rat consistent en des injections ponctuelles, alors que chez l'homme l'effet thérapeutique maximal sialagogue est obtenu par voie orale au bout de 4 à 8 semaines de traitement pour la pilocarpine (15 à 30 mg/jour), et un délai de 2 à 3 semaines peut être nécessaire pour voir apparaître les premiers effets de la yohimbine (15 à 20 mg/jour). Cette association permet en outre de diminuer les posologies et de réduire le risque d'effets indésirables.

Ainsi, une composition sialagogue selon l'invention permet de soulager efficacement un individu atteint d'un défaut de la fonction salivaire, et en particulier d'une hyposialie ou d'une xérostomie.

De manière avantageuse, dans le cadre de la présente invention, l'agoniste muscarinique est choisi parmi la pilocarpine ou le chlorhydrate de pilocarpine et en ce que l'antagoniste adrénergique est choisi parmi la yohimbine ou le chlorhydrate de yohimbine ; la composition comprend en outre un composé sialagogue complémentaire ; le composé sialagogue complémentaire est choisi parmi *Anethum graveolens* L. Asteraceae, *Arctium lappa* L. Asteraceae, *Areca catechu* L. Palmae, *Capsicum spp.* Solanaceae, *Carlina acaulis* L. Asteraceae, *Centaurium erythraea* Rafn. Gentianaceae, *Cephaelis spp*. Rubiaceae, *Cinchona spp.* Rubiaceae, *Cnicus benedictus* L. Asteraceae, *Cochlearia spp*. Brassicaceae, *cytisus scoparius* L. Link. Fabaceae, *Echinaceae spp.* Asteraceae, *Erysimum officinale* L. Brassicaceae, *Foeniculum spp*. Apiaceae, *Gentiana spp*. Gentianaceae, *Glycyrrhiza glabra* L. Fabaceae, *Harpagophytum procumbens* Burch. DC. ex Meissn. Pedaliaceae, *Humulus lupulus* L. Cannabaceae, *Huperzia serrata* Thunb. Trevis. Lycopodiaceae, *Illicium verum* Hook. *f.* Illiciaceae, *Inula helenium* L. Asteraceae, Laburnum *anagyroides* Medikus Fabaceae, *Lamium album* L. Lamiaceae, *Lippia citriodora* H.B. et K. Verbenaceae, *Lupinus spp.* Fabaceae, *Maytenus* illicifolia Mart ex Reissek Celastraceae, *Medicago sativa* L. Fabaceae, *Melaleuca* spp. Myrtaceae, *Mentha* spp. Lamiaceae, *Menyanthes trifoliata* L. Menyanthaceae, *Myrica cerifera* L. Myricaceae, *Olea europea* L. Oleaceae, *Panax ginseng* spp. Araliaceae, *Petroselinum crispum* Mill. A.W. Hill. Apiaceae, *Peucedanum ostruthium* L. Koch Apiaceae, *Picrasma excelsa* Sw. Planch. Simaroubaceae, *Pimpinella anisum* L. Asteraceae, *Piper spp.* Piperaceae, Polygala *spp*. Polygalaceae, *Primula veris* L. Primulaceae, Quassia *amara* L. Simaroubaceae, Raphanus *sativus* L. var. niger Mill. Kerner Brassicaceae, Rheum spp. Polygonaceae, Tanacetum *cinerariifolium* Trev. Schultz Bip. Asteraceae, *Tilia* spp. Tiliaceae, *Verbena officinalis* L. Verbenaceae, *Zingiber officinalis* Roscoe Zingiberaceae, *Ziziphus* jujuba Miller Rhamnaceae, ou plantes voisines et leurs extraits, les « principes amers », la choline et ses dérivés, l'anéthole et l'anétholtrithione ; la composition est destinée à l'homme et elle convient pour une administration orale, de préférence transmuqueuse ; elle est sous forme liquide, de préférence sous forme de solution, de spray, de gouttes, sous forme semi liquide ou sous forme solide, de préférence sous forme de comprimé, de poudre, de granulés ou de biscuit ; le traitement d'un défaut de la fonction salivaire est destiné à l'homme et comprend l'hyposialie ou la xérostomie ; la composition est utilisée pour obtenir un médicament destiné au traitement d'un défaut de la fonction salivaire ; la composition est administrée en association avec un traitement local qui respecte, protège ou restaure le biofilm oral normal et qui comprend l'administration d'un composé antifongique.

L'invention sera mieux comprise à la lecture de l'exposé non limitatif qui suit.

Dans le cadre de l'invention, une composition sialogogue, ou sialagogue, est une composition apte à augmenter la sécrétion salivaire chez un individu, en particulier dans le cadre d'un traitement d'un défaut de la fonction salivaire. L'individu peut être un homme (au sens large d'humain) ou un animal, de préférence un homme.

De manière avantageuse, un test tel que défini dans la demande de brevet français n°06 11099 (PCT/FR2007/002083) est préalablement effectué chez l'individu afin de détecter la présence d'un défaut de la fonction salivaire. Ce test met en oeuvre une préparation solide telle qu'un biscuit, des granulés ou une poudre tels que définis dans ladite demande.

Les agonistes muscariniques comprennent la pilocarpine ainsi que le chlorhydrate de pilocarpine. La pilocarpine peut être obtenue à partir de plantes, par exemple Pilocarpus microphyllus Stapf, Rutaceae.

Les antagonistes adrénergiques 2 comprennent la yohimbine, pour l'obtention d'un médicament. La yohimbine peut être obtenue à partir d'une plante *Pausinystalia yohimbe* (K. Schum.) Pierre Rubiaceae.

De façon encore préférée, la composition comprend la pilocarpine et la yohimbine. En effet, la pilocarpine peut produire des effets secondaires tels que des nausées et/ou des éructations lorsqu'elle est administrée seule, tandis que, de manière surprenante, aucun effet secondaire n'est observé lorsqu'elle est administrée en association avec la yohimbine. Cela est illustré par une étude réalisée sur 5 individus volontaires sains, deux hommes (H) âgés de 15 et 19 ans, et trois femmes (F) âgées de 16, 46 et 76 ans. Ces volontaires ont absorbé, en une seule, un à trois des comprimés ci-dessous :
- R3 : pilocarpine (Salagen^{®}) 1 comprimé à 5 mg
- R4 : éséridine (Génésérine^{®} 4,5) 1 comprimé à 4,5 mg
- R5 : yohimbine (Yohimbine Houdé^{®}) 1 comprimé à 2 mg
- R7 : anéthotrithione (Sulfarlem S25^{®}) 1 comprimé à 25 mg.

L'augmentation de la sécrétion de salive chez ces volontaires a été mesurée dans chaque cas et notée de 0 à 3. Les résultats sont reportés dans le tableau ci-dessous :

| | | **Augmentation de salive, notée de 0 à 3** | | | | | | |
|---|---|---|---|---|---|---|---|---|
| | | R3 | R5 | R7 | R3+R5 | R3+R7 | R5+R7 | R3+R5+R7 |
| Cas 1 | H 19 ans | 0 | 0 | 0 | 0 | 1 | 0 | NT |
| Cas 2 | H 15ans | 2 | 0 | 0 | 2 | 0 | 0 | NT |
| Cas 3 | F 72 ans | 2 | 1 | 1 | 1 | 1 | 1 | NT |
| Cas 4 | F 16 ans | 2 | 0 | 0 | 2 | 1 | 0 | 1 |
| Cas 5 | F 46 ans | 2 | 0 | 0 | 3 | 0 | 0 | 0 |
| | moyenne | 1,6 | 0,2 | 0,2 | 1,6 | 0,6 | 0,2 | |

| | **Augmentation de salive, notée de 0 à 3** | | | | | | | |
|---|---|---|---|---|---|---|---|---|
| | R4 | R3 | R5 | R7 | R3+R5 | R3+R7 | R5+R7 | R3+R5+R7 |
| Cas 5 + R4 | 0 | 0 | 0 | 0 | 2 | 2 | 1 | 0 |

Les effets secondaires indésirables ont également été surveillés pour chaque traitement. Ils sont reportés ci-dessous:
- Pilocarpine seule : des nausées sont observées chez tous les volontaires testés (5 cas sur 5), et des éructations dans un cas (cas n°4).
- Yohimbine seule : aucun effet secondaire observé.
- Anétholtrithione, seule ou en association: des flatulences soufrées sont observées dans les cas 3 et 5.
- Pilocarpine et yohimbine : aucun effet secondaire observé.

Ces résultats montrent que l'association d'un composé antagoniste adrénergique α2 tel que la Yohimbine (Y) et d'un composé agoniste muscarinique M3 tel que la Pilocarpine (P) permet d'augmenter la sécrétion de salive sans les effets secondaires observés lorsque ces composés sont administrés séparément. Une telle association d'un antagoniste adrénergique α2 et d'un agoniste muscarinique M3, en particulier avec un mode d'administration orale, sublinguale et/ou transmuqueuse, permet en outre de réduire les doses de principes actifs nécessaires pour augmenter la sécrétion salivaire. Un microdosage des principes actifs, pilocarpine et yohimbine, est possible parce que le volume de distribution par voie locale est < 0,8 mL pour la salive de repos *(versus* 5 L de sang), voire beaucoup moins en cas de sécheresse buccale. La clairance salivaire est elle aussi, par définition, très diminuée en cas de sécheresse buccale.

Selon un mode de réalisation préféré, la composition selon l'invention comprend en outre un ou plusieurs composés sialagogues complémentaires capables d'activer les récepteurs « périphériques A », c'est-à-dire les récepteurs sensibles aux agonistes chaud, capsaïcine, froid, menthol, piquant, astringent, CO2, salés (ions sodium), acides, amers, sucrés, arômes, unami et/ou glutamate et les récepteurs « périphériques B » qui sont situés au niveau de la synapse avec la cellule musculaire lisse de type digestif et dont les agonistes comprennent l'anétholtrithione et apparentés, conformément à la physiologie de la salivation. Ces composés complémentaires peuvent être par exemple les plantes ayant des propriétés sialagogues, apéritives, stomachiques, de stimulants digestifs, telles que *Anethum graveolens* L. Asteraceae, *Arctium lappa* L. Asteraceae, Areca *catechu* L. Palmae, *Capsicum spp.* Solanaceae, *Carlina acaulis* L. Asteraceae, *Centaurium erythraea* Rafn. Gentianaceae, *Cephaelis spp.* Rubiaceae, *Cinchona spp.* Rubiaceae, *Cnicus benedictus* L. Asteraceae, *Cochlearia spp*. Brassicaceae, *Cytisus scoparius* L. Link. Fabaceae, *Echinaceae spp.* Asteraceae, *Erysimum officinale* L. Brassicaceae, *Foeniculum spp.* Apiaceae, Gentiana *spp.* Gentianaceae, *Glycyrrhiza glabra* L. Fabaceae, *Harpagophytum procumbens* Burch. DC. ex Meissn. Pedaliaceae, *Humulus lupulus* L. Cannabaceae, Huperzia serrata Thunb. Trevis. Lycopodiaceae, *Illicium verum* Hook. *f.* Illiciaceae, Inula *helenium* L. Asteraceae, *Laburnum anagyroides* Medikus Fabaceae, *Lamium album* L. Lamiaceae, *Lippia citriodora* H.B. et K. Verbenaceae, *Lupinus spp.* Fabaceae, *Maytenus illicifolia* Mart ex Reissek Celastraceae, *Medicago sativa* L. Fabaceae, *Melaleuca* spp. Myrtaceae, *Mentha* spp. Lamiaceae, *Menyanthes trifoliata* L. Menyanthaceae, *Myrica cerifera* L. Myricaceae, *Olea europea* L. Oleaceae, *Panax ginseng* spp. Araliaceae, *Petroselinum crispum* Mill. A.W. Hill. Apiaceae, *Peucedanum ostruthium* L. Koch Apiaceae, *Picrasma excelsa* Sw. Planch. Simaroubaceae, *Pimpinella anisum* L. Asteraceae, *Piper spp.* Piperaceae, Polygala *spp*. Polygalaceae, *Primula veris* L. Primulaceae, Quassia *amara* L. Simaroubaceae, Raphanus *sativus* L. var. niger Mill. Kerner Brassicaceae, *Rheum* spp. Polygonaceae, Tanacetum *cinerariifolium* Trev. Schultz Bip. Asteraceae, Tilia spp. Tiliaceae, Verbena *officinalis* L. Verbenaceae, *Zingiber officinalis* Roscoe Zingiberaceae, *Ziziphus* jujuba Miller Rhamnaceae, ou plantes voisines, et leurs extraits, les « principes amers », la choline et ses dérivés, l'anéthole et l'anétholtrithione.

De manière surprenante, la Demanderesse a mis en évidence que la composition selon l'invention est particulièrement indiquée pour augmenter la sécrétion salivaire ou pour traiter un défaut de la fonction salivaire chez des patients, en particulier des femmes, présentant des symptômes de dépression, de perte de poids, de diminution de l'appétit, de fatigue et/ou utilisant régulièrement des bains de bouche antiseptiques. La Demanderesse a également montré que la sensation subjective de bouche sèche peut être liée à la prise de médicaments susceptibles d'altérer le biofilm, appartenant à l'une des familles pharmacologiques suivantes : antibiotiques, corticoïdes et bains de bouche antiseptiques, qui interagissent avec les bactéries productrices de glycoprotéines et avec les glycoprotéines du mucus. Ces glycoprotéines ont pour fonction physiologique de piéger les molécules d'eau (salivaire, alimentaire ou de boisson), et elles constituent l'essentiel du biofilm hydraté qui protège et lubrifie les muqueuses orales saines.

De façon préférée, la composition selon l'invention est administrée en association avec un traitement local destiné à respecter, à protéger ou restaurer le biofilm buccal. De façon préférée, ledit traitement local comprend l'administration d'un composé antifongique. La composition selon l'invention et ledit traitement local peuvent être administrés soit en même temps soit en des moments différents. De préférence, ils sont administrés sur une période de traitement commune. De préférence, la composition selon l'invention comprend en outre un composé antifongique destiné à respecter, à protéger ou restaurer le biofilm buccal.

De façon préférée, le composé antifongique possède des propriétés antifongiques contre *Candida albicans* et *Candida* spp.. De façon préférée, le composé antifongique est choisi parmi un extrait végétal ayant des propriétés antifongiques, surtout contre *Candida albicans* et *Candida* spp., comme par exemple : *Arctium lappa* L. Asteraceae, *Carlina acaulis* L. Asteraceae, *Chamomilla recutica* L. Rauschert Asteraceae, *Cinnamomum spp.* Lauraceae, *Cnicus benedictus* L. Asteraceae, *Eugenia caryophyllus* (Sprengel) Bullock & S. Harrison Myrtaceae, *Gentiana spp.* Gentianaceae, *Hedera helix* L. Araliaceae, *Hydrastis canadensis* L. Ranunculaceae, *Inula helenium* L. Asteraceae, *Lamium album* L. Lamiaceae, *Maytenus illicifolia* Mart ex Reissek Celastraceae, *Melissa officinalis* L. Lamiaceae, *Polygala spp.* Polygalaceae, *Ravensara aromatica Sonnerat* Lauraceae, *Satureja montana* L. Lamiaceae, *Smilax* spp. Liliaceae, *Solidago virgaurea* L. Asteraceae, *Thymus* spp. Lamiaceae, *Tylophora asthmatica* Wight. & Arn. Asclepiadaceae, *Ziziphus jujuba* Miller Rhamnaceae, les autres Lamiaceae à huiles essentielles antifongiques, les plantes voisines, et leurs extraits. L'antifongique peut aussi être choisi parmi les antifongiques allopathiques, en particulier les polyènes tels que amphotéricine B ou nystatine, ou les azolés tels que fluconazole, itraconazole, ou autres.

La Demanderesse a également montré que la sensation de bouche sèche est liée à la prise d'anticholinergiques "durs" et au nombre de médicaments pris chaque jour, ces deux paramètres étant significativement associés.

De façon préférée, la composition selon l'invention contenant de la pilocarpine n'est pas administrée chez les patients qui reçoivent déjà un traitement par anticholinestérasiques (antiparkinsoniens du type tropatépine), ou autres anticholinergiques : oxybutynine, ipratropium bromure, cyamémazine ou autres. Elle sera administrée en respectant les contre-indications et précautions d'emploi usuelles de la pilocarpine et de la yohimbine.

Un véhicule pharmaceutiquement acceptable est un véhicule adapté pour une utilisation en contact avec les cellules des individus humains et animaux sans toxicité, irritation, réponse allergique indue et similaires. Le choix du véhicule détermine la forme de la composition et son mode d'administration.

Dans le cadre de la présente invention, le véhicule convient pour une administration périphérique de la composition chez un individu, de préférence par voie orale. De façon encore préférée, la composition convient pour une administration orale (per os), sublinguale et/ou transmuqueuse. On entend par administration périphérique un mode d'administration qui permet aux agonistes muscariniques et aux antagonistes adrénergiques d'agir principalement et en priorité sur des cibles situées à l'extérieur du cerveau, sans nécessiter que ces composés passent la barrière hémato-encéphalique. En effet, dans le cadre de l'invention, ces composés n'exercent pas leur action principale au niveau du cerveau, bien qu'il puisse arriver qu'une partie, de préférence négligeable, de ces composés administrés parvienne au niveau du cerveau. Au contraire, dans le cadre de l'invention, l'action des agonistes muscariniques et des antagonistes adrénergiques s'exerce au niveau des synapses des voies efférentes para sympathiques et sympathiques. Pour cela, le mode d'administration et/ou le véhicule doivent permettre aux composés actifs de rester aussi longtemps que possible au niveau de la bouche, et de préférence même après la déglutition de la composition.

De façon préférée, la composition selon l'invention comprendra une huile essentielle non toxique pour la peau et les muqueuses, permettant de dissoudre les principes actifs comme le chlorhydrate de pilocarpine et le chlorhydrate de yohimbine, et favorisant leur passage à travers la muqueuse buccale pour atteindre les synapses des cellules sécrétrices salivaires et des cellulaires musculaires lisses de type digestif, avec passage minimal dans la circulation générale. Ces sels d'alcaloïdes sont généralement solubles dans les huiles essentielles, et sans décomposition au dessous de 200°C. Ces huiles essentielles seront obtenues par exemple à partir *d'Artemisia dracunculus* L. Asteraceae, *Citrus* aurantium L. *ssp.* aurantium Rutaceae, *Citrus aurantium* L. var. *Dulcis Pers.* Rutaceae, *Citrus limon* L. Brum. f. Rutaceae ou *Citrus reticulata* Blanco Rutaceae, qui ont aussi des propriétés aromatisantes et sont autorisées dans l'alimentation. Il sera également possible d'utiliser de l'huile essentielle de *Rosmarinus officinalis* L. Lamiaceae , désaromatisée ou non, qui a aussi des propriétés d'additif antioxydant et de conservateur alimentaire autorisé. Les huiles essentielles de *Citrus spp.,* obtenues par expression et non par distillation pourront être soumises ou non à des températures variant de 160 à 200°C, ou être utilisées sans cuisson, selon les modes particuliers de réalisation.

A cette fin, la composition selon l'invention peut comprendre les véhicules classiquement utilisés pour une administration périphérique, de préférence orale. Le véhicule peut donner à la composition une forme liquide, par exemple une solution, un spray, des gouttes, une forme semi liquide, par exemple un gel ou une émulsion, ou une forme solide, par exemple un comprimé, un comprimé lyoc, une poudre ou un biscuit. De préférence, la composition sous forme solide est friable par la seule action de la langue d'un individu. Ainsi, l'individu qui met en bouche la composition sous forme solide peut l'émietter, par exemple en la tenant entre sa langue et son palais, sans l'intervention de ses dents, sans mastication. Les compositions sous forme de spray, de gouttes, de gel, d'émulsion, de comprimés lyoc, de pâtes, de pâtes à mâcher, de biscuit, de granulés ou de poudres sont préférées car ces formes permettent aux composés actifs de rester dans la bouche plus longtemps que d'autres formulations, notamment qu'un comprimé enrobé.

Selon un mode de réalisation préféré, la forme solide comprend un biscuit, une poudre ou des granulés tels que ceux décrits dans la demande de brevet français n°06 11099, comprenant un ou plusieurs produits céréaliers, une matière grasse et de l'oeuf. Leur fabrication peut comprendre la préparation d'un mélange comprenant de 58% à 68% de son poids en farine, de 26,5% à 36,5% de son poids en matière grasse, de préférence en huile végétale, et de 1,5% à 9,5% de son poids en blanc d'oeuf, puis la cuisson de ce mélange de 15 à 25 minutes à une température comprise entre 160°C et 200°C, puis l'obtention soit directement d'un biscuit, de poudre ou de granulés, soit d'un biscuit qui est broyé pour obtenir la poudre ou les granulés. De préférence, la matière grasse comprend l'huile d'amande douce, de l'huile d'olive ou de l'huile de sésame, en particulier lorsque la composition selon l'invention comprend du *Tanacetum cinerariifolium* ou un extrait.

La poudre et les granulés sont avantageux car ils permettent de résoudre le problème de la fragilité des biscuits, liée à leur friabilité, lors des manipulations et des transports, ainsi que la difficulté de trouver un conditionnement qui évite aux biscuits de s'émietter. La poudre ou les granulés peuvent être avantageusement conditionnés sous vide pour limiter l'oxydation de la préparation et augmenter la durée de conservation. Le conditionnement de la poudre ou des granulés, par exemple dans une seringue ou un dispositif doseur, permet de délivrer une dose précise de préparation sur la langue, et de respecter les règles d'hygiène lors de son utilisation.

De plus, un principe actif ou une huile essentielle ne supportant pas la cuisson peuvent être mélangés dans le biscuit, la poudre ou les granulés précités, par exemple en étant dissouts ou dilués dans la matière grasse les composant et/ou en étant comprimés avec le biscuit.

De façon préférée, la composition selon l'invention comprend entre 0,001 et 100 mg d'agoniste muscarinique M3 choisi parmi la pilocarpine ou le chlorhydrate de pilocarpine et entre 0,001 et 100 mg d'antagoniste adrénergique a2 choisi parmi la yohimbine ou le chlorhydrate de yohimbine choisi parmi la yohimbine ou le chlorhydrate de yohimbine. De façon plus préférée, la composition selon l'invention comprend entre 0,01 et 10 mg de pilocarpine et entre 0,01 et 10 mg de yohimbine, par exemple 5 mg de pilocarpine et 2 mg de yohimbine.

Des exemples non limitatifs de formulation sont donnés ci-après.

### Exemple 1 : Comprimé de 0,2 g:

| | |
|---|---|
| Pilocarpine chlorhydrate | 5 mg |
| Yohimbine | 2 mg |
| Amidon | 0,118 g |
| Phosphate bicalcique | 0,020 g |
| Silice | 0,020 g |
| Lactose | 0,030 g |
| Talc | 0,010 g |
| Stéarate de magnésium | 0,005 g |

## Revendications

1. Composition sialagogue comprenant, dans un véhicule pharmaceutiquement acceptable convenant pour une administration périphérique, un agoniste muscarinique M3 choisi parmi la pilocarpine ou le chlorhydrate de pilocarpine, **caractérisée en ce qu'**elle comprend en outre un antagoniste adrénergique α2 choisi parmi la yohimbine ou le chlorhydrate de yohimbine.

2. Composition selon la revendication 1, **caractérisée en ce qu'**elle comprend en outre un composé sialagogue complémentaire.

3. Composition selon la revendication 2, **caractérisée en ce que** le composé sialagogue complémentaire est choisi parmi *Anethum graveolens L*. Asteraceae, *Arctium lappa L.* Asteraceae, *Areca catechu L*. Palmae, *Capsicum spp.* Solanaceae, *Carlina acaulis* L. Asteraceae, *Centaurium erythraea Rafn.* Gentianaceae, *Cephaelis spp.* Rubiaceae, *Cinchona spp.* Rubiaceae, *Cnicus benedictus L*. Asteraceae, *Cochlearia spp.* Brassicaceae, *Cytisus scoparius L*. Link. Fabaceae, *Echinaceae spp.* Asteraceae, *Erysimum officinale L*. Brassicaceae, *Foeniculum spp*. Apiaceae, *Gentiana spp.* Gentianaceae, *Glycyrrhiza glabra L*. Fabaceae, *Harpagophytum procumbens* Burch. DC. ex Meissn. Pedaliaceae, *Humulus lupulus L*. Cannabaceae, *Huperzia serrata* Thunb. Trevis. Lycopodiaceae, *Illicium* verum Hook. f. Illiciaceae, *Inula helenium L*. Asteraceae, *Laburnum anagyroides* Medikus Fabaceae, *Lamium album L*. Lamiaceae, *Lippia citriodora* H.B. et K. Verbenaceae, *Lupinus spp.* Fabaceae, *Maytenus illicifolia* Mart ex Reissek Celastraceae, *Medicago sativa L*. Fabaceae, *Melaleuca spp*. Myrtaceae, *Mentha spp*. Lamiaceae, *menyanthes trifoliata L*. Menyanthaceae, *Myrica cerifera L*. Myricaceae, *Olea europea L*. Oleaceae, *Panax ginseng spp.* Araliaceae, *Petroselinum crispum* Mill. A.W. Hill. Apiaceae, *Peucedanum ostruthium L*. Koch Apiaceae, *Picrasma excelsa* Sw. Pianch. Simaroubaceae, *Pimpinella anisum L*. Asteraceae, *Piper spp.* Piperaceae, *Polygala spp.* Polygalaceae, *Primula veris L*. Primulaceae, *Quassia amara L*. Simaroubaceae, *Raphanus sativus L*. var. niger Mill. Kerner Brassicaceae, *Rheum spp.* Polygonaceae, *Tanacetum cineraniifolium Trev. Schuitz Bip. Asteraceae, Tilla spp. Tiiiaceae, Verbena officinalis L. Verbenaceae, Zingiber officinalis* Roscoe Zingiberaceae, *Ziziphus jujuba* Miller Rhamnaceae, ou plantes voisines et leurs extraits, les «principes amers», la choline et ses dérivés, l'anéthole et l'anétholtrithione.

4. Composition selon l'une des revendications 1 à 3, **caractérisée en ce qu'**elle est destinée à l'homme et **en ce qu'**elle convient pour une administration orale, de préférence transmuqueuse.

5. Composition selon l'une des revendications 1 à 4, **caractérisée en ce qu'**elle est sous forme liquide, de préférence sous forme de solution, de spray, de gouttes, sous forme semi liquide ou sous forme solide, de préférence sous forme de comprimé, de poudre, de granulés ou de biscuit.

6. Composition selon l'une des revendications 1 à 5 pour le traitement d'un défaut de la fonction salivaire.

7. Composition selon la revendication 6 **caractérisée en ce que** ledit traitement est destiné à l'homme, et **en ce que** le défaut de la fonction salivaire comprend l'hyposialie ou la xérostomie.

8. Utilisation d'une composition selon l'une des revendications 1 à 5 pour l'obtention d'un médicament destiné au traitement d'un défaut de la fonction salivaire.

9. Utilisation selon la revendication 8 **caractérisée en ce que** ledit traitement est destiné à l'homme, et **en ce que** le défaut de la fonction salivaire comprend l'hyposialie ou la xérostomie.

10. Utilisation selon l'une des revendications 8 à 9, **caractérisée en ce que** la composition est associée à un composé antifongique à action locale.

## Patentansprüche

1. Den Speichelfluss anregendes Stoffgemisch, umfassend, in einem pharmazeutisch akzeptablen Vehikel, das für die periphere Verabreichung geeignet ist, einen Muskarin-M3-Agonisten, ausgewählt aus dem Pilocarpin oder dem Pilocarpinhydrochlorid, **dadurch gekennzeichnet, dass** es außerdem einen α2-adrenergen Antagonisten umfasst, ausgewählt aus dem Yohimbin oder dem Yohimbinhydrochlorid.

2. Stoffgemisch nach Anspruch 1, **dadurch gekennzeichnet, dass** es außerdem ein zusätzliches den Speichelfluss anregendes Stoffgemisch umfasst.

3. Stoffgemisch nach Anspruch 2, **dadurch gekennzeichnet, dass** das zusätzliche den Speichelfluss anregende Stoffgemisch ausgewählt ist aus *Anethum graveolens L. Asteraceae, Areca catechu L. Palmae, Capsicum spp. Solanaceae, Carlina acaulis L. Asteraceae, Centaurium erythraea Rafn. Gentianaceae, Cephaelis spp. Rubiaceae, Cinchona spp. Rubiaceae, Cnicus benedictus L. Asteraceae, Cochlearia spp. Brassicaceae, Cytisus scoparius L. Link. Fabaceae, Echinaceae spp. Asteraceae, Erysimum officinale L. Brassicaceae, Foeniculum spp. Apiaceae, Gentiana spp. Gentianaceae, Glycyrrhiza glabra L. Fabaceae, Harpagophytum procumbens Burch. DC. ex Meissn. Pedaliaceae, Humulus lupulus L. Cannabaceae, Huperzia serrata Thunb. Trevis. Lycopodiaceae, Illicium verum Hook. f. Illiciaceae, Inula helenium L. Asteraceae, Laburnum anagyroides Medikus Fabaceae, Lamium album L. Lamiaceae, Lippia citriodora H.B. et K. Verbenaceae, Lupinus spp. Fabaceae, Maytenus illicifolia Mart ex Reissek Celastraceae, Medicago sativa L. Fabaceae, Melaleuca spp. Myrtaceae, Mentha spp. Lamiaceae, Menyanthes trifoliata L. Menyanthaceae, Myrica ceri fera L. Myricaceae, Olea europea L. Oleaceae, Panax ginseng spp. Araliaceae, Petroselinum crispum Mill. A.W. Hill. Apiaceae, Peucedanum ostruthium L. Koch Apiaceae, Picrasma excelsa Sw. Pianch. Simaroubaceae, Pimpinella anisum L. Asteraceae, Piper spp. Piperaceae, Polygala spp. Polygalaceae, Primula veris L. Primulaceae, Quassia amara L. Simaroubaceae, Raphanus sativus L. var. niger Mill. Kerner Brassicaceae, Rheum spp*. *Polygonaceae, Tanacetum cineraniifolium Trev. Schuitz Bip. Asteraceae, Tilla spp. Tiiiaceae, Verbena officinalis L. Verbenaceae*, *Zingiber officinalis Roscoe Zingiberaceae, Ziziphus jujuba Miller Rhamnaceae,* oder ähnliche Pflanzen und ihre Extrakte, die "bitteren Stoffe", das Cholin und seine Derivate, das Anethol und das Anetholtrithion.

4. Stoffgemisch nach einem der Ansprüche 1 bis 3, **dadurch gekennzeichnet, dass** es für den Menschen bestimmt ist und dadurch, dass es für eine orale, vorzugsweise transmucosane Verabreichung geeignet ist.

5. Stoffgemisch nach einem der Ansprüche 1 bis 4, **dadurch gekennzeichnet, dass** es in flüssiger Form vorliegt, vorzugsweise in Form einer Lösung, eines Sprays, von Tropfen, in halbflüssiger oder in fester Form, vorzugsweise in Form einer Tablette, eines Puders, von Körnchen oder eines Biskuits.

6. Stoffgemisch nach einem der Ansprüche 1 bis 5 für die Behandlung eines Defekts der Speichelfunktion.

7. Stoffgemisch nach Anspruch 6, **dadurch gekennzeichnet, dass** die Behandlung für den Menschen bestimmt ist, und dadurch, dass der Defekt der Speichelfunktion die Hyposialie oder die Xerostomie umfasst.

8. Verwendung eines Stoffgemischs nach einem der Ansprüche 1 bis 5 zum Erhalt eines Arzneimittels, das für die Behandlung eines Defekts der Speichelfunktion bestimmt ist.

9. Verwendung nach Anspruch 8, **dadurch gekennzeichnet, dass** die Behandlung für den Menschen bestimmt ist, und dadurch, dass der Defekt der Speichelfunktion die Hyposialie oder die Xerostomie umfasst.

10. Verwendung nach einem der Ansprüche 8 bis 9, **dadurch gekennzeichnet, dass** das Stoffgemisch mit einer antimykotischen Verbindung mit lokaler Wirkung assoziiert ist.

## Claims

1. Sialogogue composition comprising, in a pharmaceutically acceptable vehicle suitable for peripheral administration, an M3 muscarinic agonist chosen from pilocarpine or pilocarpine hydrochloride, **characterised in that** it further comprises an α-2 adrenergic antagonist chosen from yohimbine or yohimbine hydrochloride.

2. Composition according to claim 1, **characterised in that** it further comprises an additional sialogogue compound.

3. Composition according to claim 2, **characterised in that** the additional sialogogue compound is chosen from *Anethum graveolens L.* Asteraceae, *Arctium lappa L.* Asteraceae, *Areca catechu L.* Palmae, *Capsicum spp.* Solanaceae, *Carlina acaulis L.* Asteraceae, *Centaurium erythraea Rafn.* Gentianaceae, *Cephaelis spp.* Rubiaceae, *Cinchona spp.* Rubiaceae, *Cnicus benedictus L.* Asteraceae, *Cochlearia spp.* Brassicaceae, *Cytisus scoparius L.* Link. Fabaceae, *Echinaceae spp.* Asteraceae, *Erysimum officinale L*. Brassicaceae, *Foeniculum spp.* Apiaceae, *Gentiana spp.* Gentianaceae, *Glycyrrhiza glabra L.* Fabaceae, *Harpagophytum procumbens* Burch. DC. ex Meissn. Pedaliaceae, *Humulus lupulus L.* Cannabaceae, *Huperzia serrata* Thunb. Trevis. Lycopodiaceae, *IIlicium verum* Hook. f. Illiciaceae, *Inula helenium L.* Asteraceae, *Laburnum anagyroides* Medikus Fabaceae, *Lamium album L.* Lamiaceae, *Lippia citriodora* H.B. and K. Verbenaceae, *Lupinus spp.* Fabaceae, *Maytenus illicifolia* Mart ex Reissek Celastraceae, *Medicago sativa L.* Fabaceae, *Melaleuca spp.* Myrtaceae, *Mentha spp.* Lamiaceae, *Menyanthes trifoliata L.* Menyanthaceae, *Myrica cerifera L.* Myricaceae, *Olea europea L.* Oleaceae, *Panax ginseng spp.* Araliaceae, *Petroselinum crispum* Mill. R.W. Hill. Apiaceae, *Peucedanum ostruthium L.* Koch Apiaceae, *Picrasma excelsa* Sw. Pianch. Simaroubaceae, *Pimpinella anisum L.* Asteraceae, *Piper spp.* Piperaceae, *Polygala spp.* Polygalaceae, *Primula veris* L. Primulaceae, *Quassia amara* L. Simaroubaceae, *Raphanus sativus L.* var. niger Mill. Kerner Brassicaceae, *Rheum spp.* Polygonaceae, *Tanacetum cineraniifolium Trev. Schuitz Bip. Asteraceae, Tilla spp. Tiliaceae, Verbena officinalis L. Verbenaceae, Zingiber officinalis* Roscoe Zingiberaceae, *Ziziphus jujube* Miller Rhamnaceae, or closely related plants and extracts thereof, "bitter principles", choline and derivatives thereof, anethole and anethole trithione.

4. Composition according to any of claims 1 to 3, **characterised in that** it is intended for humans and **in that** it is suitable for oral, preferably transmucosal, administration.

5. Composition according to any of claims 1 to 4, **characterised in that** it is in liquid form, preferably in the form of solution, spray, drops, in semi-liquid form or in solid form, preferably in tablet, powder, granule or biscuit form.

6. Composition according to any of claims 1 to 5 for treating a salivary function defect.

7. Composition according to claim 6, **characterised in that** said treatment is intended for humans, and **in that** the salivary function defect comprises hyposialiosis or xerostomia.

8. Use of a composition according to any of claims 1 to 5 for obtaining a medicinal product intended for treating a salivary function defect.

9. Use according to claim 8 **characterised in that** said treatment is intended for humans, and **in that** the salivary function defect comprises hyposialiosis or xerostomia.

10. Use according to any of claims 8 to 9, **characterised in that** the composition is associated with a locally acting antifungal compound.
